Europäisches Patentamt

European Patent Office

Office européen des brevets

(11) Publication number: **0 277 043**
**A2**

## (12) EUROPEAN PATENT APPLICATION

(21) Application number: 88300803.9

(22) Date of filing: 29.01.88

(51) Int. Cl.⁴: **C 07 G 15/00**
C 07 K 3/24, A 61 K 37/36

(30) Priority: 30.01.87 US 9291

(43) Date of publication of application:
03.08.88 Bulletin 88/31

(84) Designated Contracting States:
AT BE CH DE ES FR GB GR IT LI LU NL SE

(71) Applicant: INTERNATIONAL MINERALS AND CHEMICAL
CORPORATION
P.O. Box 207 1401 South Third Street
Terre Haute Indiana 47808 (US)

(72) Inventor: Janski, Alvin M.
1132 Whitfield Road
Northbrook Illinois 60062 (US)

Martin, Jerome L.
1334 South Center Street
Terre Haute Indiana 47802 (US)

Atkinson, Paul R.
1321 Washington Street Apt. 2D
Evanston Illinois 60202 (US)

(74) Representative: Holmes, Michael John et al
Frank B. Dehn & Co. Imperial House 15-19 Kingsway
London WC2B 6UZ (GB)

(54) Method for recovering somatotropins from dilute aqueous solution.

(57) A method for the recovery of somatotropin, in particular a fragment of somatotropin, from dilute aqueous solutions comprising adding to the aqueous solution a salt of transition metal to form an insoluble complex with the somatotropin.

The insoluble complex may be separated from the solution, by either centrifugation or filtration, and dried by conventional methods such as lyophilization or removal of water at low temperatures under vacuum. The method results in the recovery of a dried bioactive somatotropin.

EP 0 277 043 A2

**Description**

## METHOD OF RECOVERING SOMATOTROPINS FROM DILUTE AQUEOUS SOLUTIONS

Field of the Invention

This invention relates to a process for recovering bioactive proteins from dilute aqueous solutions. More specifically, this invention relates to a process for recovering bioactive somatotropin from aqueous solutions by the addition of transition metal salts to the solutions.

Background of the Invention

Somatotropins, also known as growth hormones, are polypeptide hormones secreted by the pituitary glands of many animal species. These hormones are valuable for a number of therapeutic uses, and compositions comprising somatotropins can be administered in the treatment of pituitary deficiency in humans and gastrointestinal bleeding or to promote the healing of bone fractures and accelerate the healing of contusions and other wounds. Somatotropins also are useful in promoting meat and milk production in animals when administered through various drug-releasing devices or by injection. (See E.J. Turman, "Some Effects of Pituitary Anterior Growth Factor" Thesis: Purdue University, April, 1953; L.J. Machlin J. Anim. Sci. 35: 794-800 (1972); T.R. Kasser et al." J. Anim. Sci. 53: 420-426 (1981); L.J. Machlin, J. Dairy Sci. 56: 575-580 (1973)). Although somatotropins are somewhat species specific, there is considerable homology among the amino acid sequences of animal somatoropins and these hormones have been shown to exhibit inter-species activity. In addition, various active fragments of somatotropins have been discovered.

Traditionally, somatotropins have been obtained by isolation from excised pituitary tissue. With the advent of recombinant DNA technology, it has become possible to obtain somatotropins from genetically engineered microorganisms containing recombinant DNA which specifies the production of somatotropin. See, for example, European Patent Application 83304574.3 (publication number 0 103 395) to Biogen N.V. Whether the somatotropin's source is animal or microbial, purification is required to remove contaminants such as other proteins, polypeptides, and cellular debris and to provide the protein in its properly folded, bioactive form. The somatotropin may be purified using one or more known methods of protein purification including gel chromatography, affinity chromatography, ion-exchange chromatography, ultrafiltration, dialysis, precipitation with salts such as ammonium sulfate, extraction from inclusion bodies using guanidine-HCl or sodium dodecyl sulfate, and many other known techniques. Examples of some of the various purification techniques for somatotropin may be found in the Kirk-Othmer Encyclopedia of Chemical Technology, 3rd Edition, Volume 11; U.S. Patent No. 4,371,462, issued to Hecht; and Hart, et al., Biochem J., 218:573-581 (1984).

The result of many of these purification processes is a dilute aqueous solution containing somatotropin. Thus, a recovery process is needed to reclaim purified somatotropin from these dilute aqueous solutions.

Current methods for recovery of matotropin involve freeze-drying a dilute solution of the hormone. However, lyophilization is a costly process for recovering dried biologically active materials due to the low rate of production, the high cost of equipment and the necessity of maintaining very low pressures during sublimation of the water substance from the frozen state. Lyophilization is especially cost intensive on a large scale because of the large volumes of solution involved.

A process is needed in the art which is less costly and does not adversely affect the bioactivity of the somatotropin.

An object of the present invention is to provide an inexpensive method of recovering somatotropin from aqueous solutions without lessening its bioactivity.

Another object of the present invention is to provide such a method which would lower equipment costs in comparison to equipment costs of conventional recovery methods.

A further object of the present invention is to provide a method of recovering somatotropin from aqueous solutions which would lower labor costs in comparison to the cost of labor by conventional recovery methods.

SUMMARY OF THE INVENTION

In accordance with the present invention, there is disclosed a method of recovering somatotropin from dilute aqueous solutions comprising adding to the aqueous solutions salts of transition metals to form insoluble complexes with the somatotropin.

The insoluble complexes may be separated from the solution, as by either centrifugation or filtration, followed by drying by either lyophilization, removal of water at low temperatures under vacuum or by other procedures. The method results in the recovery of a dried bioactive somatotropin.

DETAILED DESCRIPTION OF THE INVENTION

This invention is directed to a method for recovering somatotropin (hereinafter also referred to as ST) from dilute aqueous solutions resulting from the purification of somatotropin from a pituitary homogenate or a fermentation medium. The method of this invention may be used to recover various types of somatotropins from dilute aqueous solutions, including natural or recombinant porcine, bovine, ovine, human, gallinaceous fowl, or fish.

As used herein, the term somatotropin is intended to include the full length natural of recombinant somatotropin as well as derivatives thereof that have growthpromoting capabilities. Derivatives include biologically active fragments of the polypeptide hormone; such fragments are, in fact, preferred forns of somatotropin for use in accordance with the invention. Particularly preferred fragments include the $\Delta 4$ construction of bovine somatotropin (a polypeptide missing 4 amino acids from the Nterminal, described in the Biogen European Patent Application.mentioned above), and a polypeptide designated $\Delta 7$ porcine somatotropin, which has an amino acid sequence corresponding to that of porcine somatotropin, less the first seven amino acids of the mature, full length hormone (and is described in European Patent Application Publication No. 0 104 920 to Biogen N.V.). The term somatotropin also includes an active fragment of the polypeptide that comprises an extraneous N-terminal methionine.

The term "biologically active" as used herein means a polypeptide that, following its administration to a living being has a demonstrable effect on a biological process of that living being. A biologically active somatotropin can enhance the growth rate of a living being when administered to that living being in growth-enhancing amounts. A biologically active somatotropin also can enhance the feed efficiency, nutrient distribution or carcass quality of a living being when administered to that living being in appropriate amounts.

The method of this invention comprises the addition of salts of transition metals to a dilute aqueous solution containing the somatotropin. Desirably, the somatotropin in solution is in its properly folded, bioactive form. The salts of the transition metals form insoluble complexes with the somatotropin, thus precipitating the somatotropin out of the dilute aqueous solutions. The complexes comprise the somatotropin molecules and metal ions such as $Zn^{2+}$, $Cu^{2+}$, $Co^{2+}$, $Mn^{2+}$, $Fe^{2+}$ or $Fe^{3+}$. These Complexes contain ligand bonds between the metal ion and the nitrogen atoms of some of the amino acid residues in the somatotropin molecule. Following precipitation of the somatotropin-metal complex, the precipitate may be separated from most of the aqueous medium in the for:n of a concentrated aqueous slurry. The insoluble complexes then may be dried to remove the remaining water. The resultant product is a dried somatotropin transition metal complex. The presence of the transition metal in the product has been shown to have no significant adverse effect on the bioactivity of the somatotropin when the product is administered to a living being.

As noted above, in accordance with the conventional procedures for obtaining a somatotropin, solutions of natural somatotropin in a pituitary homogenate or recombinant somatotropin in a fermentation medium contain large amounts of water in comparison to the amount of somatotropin. Such solutions can contain, for example, about 100 grams of water per gram of somatotropin or more. In accordance with the conventional recovery methods the entire amount of water is subjected to lyophilization. However, when the method of this invention is used and a transition metal salt is added to the solution to precipitate the somatotropin out of solution as a somatotropin-transition metal complex, most of the initial large volume of water can be separated easily from the precipitate, leaving a concentrated aqueous slu.rry to be dried. Using the 100:1 ratio of water to somatotropin in the initial solution set forth above as an example, the ratio of water to somatotropin in the concentrated slurry typically may be only 5 grams:1 gram. Thus, the load of water to be removed has been reduced by a factor of twenty fold.

Furthermore, the water associated with the bioactive complex of somatotropin with a transition metal ion can be removed much faster with less costly equipment than that used for lyophilization. The process of the invention provides very substantial savings in capital for equipment and buildings as well as in labor for operating the drying process.

The transition metals which may be used in the process of this invention include zinc, copper, manganese, iron and cobalt. The preferred metals include zinc, copper, and manganese; most preferred is zinc. Salts of these metals which are especially effective in the method of the present invention include the chlorides of zinc, copper, manganese, iron, and cobalt, but other salts, such as sulfates, acetates, or tartrates, also may be used in the process.

The method of this invention is useful for recovering somatotropin from dilute aqueous solutions which result from various purification processes. The somatotropin which may be recovered through the method of this invention may be obtained by isolation from excised pituitary tissue or genetically engineered microorganisms containing recombinant DNA which specifies the production of somatotropin.

The aqueous solutions from which somatotropin is to be recovered may contain varying amounts of somatotropin. The transition metal salt is added in an amount effective to precipitate the somatotropin. Generally, in laboratory-scale reactions, the metal salt can be added such that its concentration ranges from about 0.12 mmoles to about 12 mmoles per 0.025 mmoles of somatotropin in 1.0 liter of solution. A typical amount of metal used in such reactions can be about 1.2 mmoles of metal salt per 0.025 mmoles somatotropin in 1.0 liter of aqueous solution. The amount of transition metal salt sufficient to effectively precipitate the somatotropin can vary, however. In the large scale reaction of example X of this application, for instance, it was found that a ratio of about 10 moles of zinc salt to 1 mole of recombinant porcine ST was effective to precipitate the ST. Effective amounts can be determined by routine experimentation by persons with ordinary skill in the art. Usually, an excess of the metal salt will be employed for complete precipitation of the somatotropin in the solution. The final product of somatotropintransition metal salt complex generally contains at least about 1-8 moles metal/one mole ST. On a weight percent basis, the product generally contains between 0.3 and 8 percent by weight transition metal. Preferably, the product contains about 0.4 to about 7 weight percent metal.

The precipitation of the somatotropin preferably is carried out in near neutral or basic solutions. The pH of the aqueous solution desirably is between 6.8 and 9.8. The dilute aqueous solutions of somatotropin will most preferably be at a pH of between about 7.4 and 9.0 for efficient recovery of the somatotropin.

Various buffers may be used in the aqueous solutions containing somatotropin. The buffers are added to help maintain the somatotropin in solution at a given pH prior to the addition of the transition metal salt. Buffers containing anions that form insoluble salts with metal ions $Zn^{2+}$, $Cu^{2+}$, $Co^{2+}$. $Mn^{2+}$, $Fe^{2+}$, or $Fe^{3+}$ should be used with caution. Useful buffers for the present 5 method include carbonate buffer (0.42 mM $Na_2CO_3$, 0.50mM $NaHCO_3$, adjusted to a pH between 7.4 and 9.8); 50 mM Tris HCl at about pH 7.4; and 60 mM ethanolamine at about pH 9.8.

The addition of the transition metal salt to 10 dilute aqueous somatotropin-containing solution causes the precipitation of a metal-somatotropin complex. The solution desirably is stirred while the metal-sbmatotropin complex precipitates out of the aqueous solution.

After the salt of the transition metal has been added to the dilute aqueous somatotropin-containing solution and the somatotropin has precipitated out as an insoluble somatotropin-metal salt complex, the insoluble complexes then may be separated from the aqueous solutions and dried. The separation step may be in accordance with conventional procedures, such as centrifugation or filtration, or a combination of both procedures. The precipitated material may be pelleted by centrifugation at room temperature or separated by filtration and then dried by a conventional method, such as removal of water at low temperatures under a vacuum or lyophilization. If the centrifuged or filtered somatotropin is to be dried at low temperatures under a vacuum, the temperature may be between 0.C and 25 C.

The dried somatotropin which results from the method of this invention has substantially the same amount of bioactivity as somatotropin prepared by lyophilization or as natural somatotropin. Additionally, in vitro experiments performed to simulate in vivo administration of recombinant porcine somatotropin showed that solubility maintenance (in the presence of EDTA) was improved when the somatotropin was recovered using the method of this. invention in comparison to the recovery procedure using conventional lyophilization.

The following examples are merely illustrative of the present invention and should not be considered as limiting its scope.

## EXAMPLE I

### Precipitation of Recombinant Porcine Somatotropin

Purified lyophilized a7 recombinant porcine somatotropin (Δ7rpST), International Minerals and Chemial Corporation, lot number CF008-0BA.94.1, was reconstituted in de-ionized $H_2O$ ($dH_2O$) to a concentration of 10 mg Δ7rpST per ml. The material was dialyzed against > 100 volumes of carbonate buffer (0.42 mM $Na_2CO_3$; 0.50 mM $NaHCO_3$, adjusted to a pH of 7.4 with HCl) and a portion of this dialyzed material was diluted in nine parts of carbonate buffer, pH 7.4. The two resultant solutions of Δ7rpST (10.0 and 1.0 mg Δ7rpST/ml) were spiked with $^{125}I$-Δ7rpST (same IMC lot number) to a final concentration of 0.02μ Curie per ml to allow determination of the percentage precipitation by measurement of the radioactivity in the supernatant fraction following centrifugation.

The transition metal salts chosen for this precipitation experiment included zinc chloride, manganese chloride and cupric chloride. 24 mM, 2.4 mM and 0.24 mM solutions of each salt were made. Trichloroacetic acid (20%) was used as the positive precipitation control. The metal salts or trichloroacetic acid slutions (0.5 ml) were added to the aqueous solutions containing the a7rpST (0.5 ml) somatotropin and the precipitation step was carried out for one hour at room temperature. The precipitated material was pelleted by centrifugation at 15,000 × g for 10 minutes at room temperature. Single 0.5 ml aliquots for supernatant were removed from duplicate tubes and counted in polypropylene tubes. The minimum number of counts was four times over background. Pellet formation was judged visually and good correlation was shown between pellet size and the percent of $^{125}I$-Δ7rpST pelleted. This indicated that the $^{125}I$-Δ7rpST behaved similarly to the unlabelled Δ7rpST.

Table I illustrates the results of the experiments. At 0.5 mg Δ7rpST/ml and 5 mg Δ7rpST/ml, both zinc chloride and cupric chloride exhibited good precipitation properties at one or more reagent concentrations. Increasing the amount of metal salt resulting in equal or increasing Δ7rpST precipitation, except in the case of cupric chloride.

## TABLE I

| Precipitating Agent | Concentration | Temp. | Percent $^{125}$ Pelleted[a] | |
| --- | --- | --- | --- | --- |
| | | | 5 mg/ml rpST | 0.5 mg/ml rpST |
| Metals | | | | |
| Zinc Chloride | 12 mM | RT* | 76 ± 0.38 | 77 ± 0.96 |
| | 1.2 mM | RT | 76 ± 1.5 | 74 ± 0.56 |
| | 0.12 mM | RT | 55 ± 0.41 | 61 ± 0.21 |
| Manganese Chloride | 12 mM | RT | 27 ± 1.2 | 21 ± 1.6 |
| | 1.2 mM | RT | 22 ± 0.31 | 19 ± 2.4 |
| | 0.12 mM | RT | 8.7 ± 0.64 | 15 ± 2.0 |
| Cupric Chloride | 12 mM | RT | 23 ± 4.6 | 17 ± 2.9 |
| | 1.2 mM | RT | 64 ± 8.7 | 44 ± 6.7 |
| | 0.12 mM | RT | 42 ± 0.11 | 67 ± 0.12 |
| Controls | | | | |
| None | -- | RT | 4.3 ± 0.89 | 13 ± 1.2 |
| | -- | 4°C | 7.2 ± 2.1 | 18 ± 0.80 |
| Trichloro- acetic acid | 10% solution | RT | 98 ± 0.33 | 96 ± 0.59 |
| | 10% solution | 4°C | 98 ± 0.24 | 97 ± 0.26 |

[a]Data represents means ± std. dev., where n = 2.
*RT = room temperature

EXAMPLE II

Effect of Different Buffer and pH Conditions on the Precipitation of Δ7rpST

Purified lyophilized Δ7rpST (same lot number as in Example I) was reconstituted in $dH_2O$ to a concentration of 10 mg ml and aliquots were dialyzed against one of the following buffers: (1) carbonate buffer, pH 7.4, (2) 40 mM Tris HCl, pH 7.4, or (3) 60 mM ethanolamine, pH 9.8. The three samples were spiked with $^{125}I$-Δ7rpST as in Example I. Each sample was diluted 1:1 with a solution of 24 mM $ZnCl_2$ and the percentage precipitation of Δ7rpST was determined as in Example I. Table II illustrates the results of the experiment. The data indicate that zinc is capable of efficient precipitation of Δ7rpST from a variety of buffers at different pH's.

## Table II

Percentage Precipitation of $^{125}I$-$\Delta$7rpST from a Solution Comprised of Equal Volumes of 10 mg per ml $\Delta$7rpST and 24 mM $ZnCl_2$ in the Presence of (1) Carbonate Buffer, pH 7.4, (2) 50 mM Tris, pH 7.4, or (3) 60 mM Ethanolamine.

| Buffer | pH | Percentage 125I-$\Delta$7rpST Pelleted (a) |
|--------|-----|-----------------------------|
| carbonate | 7.4 | 76+/- 0.38 |
| 50 mM tris | 7.4 | 81+/- 0.11 |
| 60 mM ethanolamine | 9.8 | 90+/- 0.52 |

(a) Data represent means +/0 Std. Dev., where n=2.

EXAMPLE III

Solubility and Bioactivity of Precipitated $\Delta$7rpST

In preparation for the precipitation experiment, 500 mg of $\Delta$7rpST (same lot number as in Example I) was reconstituted in 50 ml of de-ionized water ($dH_2O$) resulting in a pH of 10.3 from residual carbonate ions. The material was extensively dialyzed against carbonate buffer at about pH 7.4 until the pH of the dialysate was 7.6. Volume gain was 5 ml during dialysis. The pH of the dialysate was lowered to 7.4 with 3 mEq. HCl and the solution was clarified by a 1500 × g centrifugation at room temperature.

Ten milliliter aliquots of the neutral $\Delta$7rpST solution were combined with an equal volume of either 2.4 mM $ZnCl_2$ or 2.4 mM $CuCl_2$. After one hour of stirring at room temperature, the suspensions were pelleted by a 30 minute, 15,000 × g centrifugation. Each pellet was frozen at -80.C and lyophilized along with a shell frozen control sample composed of 20 ml of the neutral pH $\Delta$7rpST solution diluted in 20 ml $dH_2O$.

The dry weight and the percentage protein purities (as measured by the BCA Protein Assay, Pierce Chemical Co., Rockford, Illinois) of the lyophilized samples were determined to allow calculation of the efficiency of each precipitation method relative to the non-precipitated, lyophilized treatment. Table III shows the results of these determinations.

## TABLE III

| Precipitation Method | Percentage $\Delta$7rpST(w/w) | Dry Weight (mg) | Weight $\Delta$7rpST (mg) | Relative Precipitation Efficiency |
|--------|--------|--------|--------|--------|
| $ZnCl_2$ | 115 | 54.5 | 62.7 | 81 |
| $CuCl_2$ | 105 | 48.0 | 50.4 | 65 |
| Lyophilization | 118 | 65.7 | 77.5 | 100 |

To determine the effect of the metal salts on the bioactivity of $\Delta$7rpST, a growth assay (determination of body weight gain) was performed using hypophysectomized rats. . See Parlow, S.F., et al., Endocrinology 77:1126-1134 (1965). The test was conducted to show the bioactivity of the somatotropin by measuring the growth promoting activity in rats of a dosage of $\Delta$7rpST recovered in accordance with the method of this invention.

To conduct the test young (36 days old at the beginning of the test period), female, hypophysectomized rats were divided into groups of 10 rats. The rats were grouped by weight, the weight and distribution of the groups was balanced such that the mean starting weight of each group was nearly equal. Each group of rats was assigned a treatment.

The rats were injected subcutaneously for each of nine days with either a somatotropin or control solution. The injections were made below the skull near the subscapular region. To solubilize somatotropin the following Parlow's buffer solutions were used.

I. $NaHCO_3$ (0.03M), NaCl (0.15M), pH raised to 10.8 with 8M NaOH.

II. $NaHCO_3$ (0.03M), NaCl (0.15M), pH raised to 9.5 with 8M NaOH.

A known amount of the Δ7rpST (see Table III) was dissolved in pH 10.8 buffer, adjusted to pH 9.5 with 2N HCl and made to volume with pH 9.5 buffer.

One group of 10 rats was used as a negative control and injected with the vehicle used in solubilizing the somatotropin. A second group was injected with a solution of lyophilized Δ7rpST. The other groups were injected with solutions of a selected dose of transition metal-precipitated Δ7rpST of this invention. The rats were weighed on days 1, 2, 9 and 10 and their body weights recorded. At the end of the test period (day 10) the rat body weight gain data was analyzed.

The growth assay with hypophysectomized rats (single 14 μg daily dose) indicated that $ZnCl_2$-precipitated Δ7rpST had significantly higher ($P < 0.01$) growth promoting activity than the negative control group and its activity was indistinguishable from that of the lyophilized Δ7rpST control that contained no transition metal salts. Similar results were observed for precipitation with cupric chloride.

TABLE IV

| Sample | Dose (μg) | Percentage Mean | ± | Wt. Gain S.D. |
|---|---|---|---|---|
| Neg. Control | 0 | 4.0 | | 4.2 |
| Δ7rpST $ZnCl_2$ ppt | 24 | 18.6* | | 1.7 |
| Δ7rpST $CuCl_2$ ppt | 24 | 15.6* | | 4.7 |
| Δ7rpST lyophilized | 24 | 17.1* | | 2.4 |

*Significantly higher ($P < 0.01$) from negative control using a One-sided Dunnett's test following an analysis of variance.

EXAMPLE IV

Precipitation of A4 Recombinant Bovine Somatotropin

Purified lyophilized Δ4 recombinant bovine somatotropin (Δ4rbST), is reconstituted in deionized $H_2O$ ($dH_2O$) to a concentration of 10 mg Δ4rbST per ml. The material is dialyzed against > 100 volumes of carbonate buffer (as defined in Example I) and a portion of this material is diluted in nine parts of carbonate buffer, pH 7.4. The resultant solutions then are spiked with $^{125}$I-Δ4rbST to a final concentration of 0.02μ Curie per ml to allow determination of the percentage precipitation by measurement of the radioactivity in the supernatant fraction following centrifugation.

The transition metal salts used include zinc chloride, manganese chloride and cupric chloride. 24 mM, 2.4 mM and 0.24 mM solutions of each salt are made. Trichloroacetic acid (20%) is used as the positive precipitation control. The metal salts or trichloroacetic acid solutions (0.5 ml) are added to the aqueous solutions containing Δ4rbST (0.5 ml) and the precipitation step is carried out with stirring for one hour at room temperature. The precipitated material is pelleted by centrifugation at 15,000 × g for 10 minutes at room remperature. Single 0.5 ml aliquots of supernatant are removed from duplicate tubes and counted in polypropylene tubes. The minimum number of counts is four times over background. Pellet formation is judged visually. The results of the experiments are similar to those in Example I.

EXAMPLE V

Solubility and Bioactivity of Precipitated Δ4rbST

In preparation for the precipitation experiment, 500 mg of Δ4rbST prepared as in Example III is reconstituted in 50 ml of de-ionized water ($dH_2O$). The material is extensively dialyzed against carbonate buffer, pH 7.4, until the pH of the dialysate is 7.6. The pH of the dialysate is lowered to 7.4 with 3mEq. HCl and the solution is clarified by a 1500 × g centrifugation at room temperature.

Ten milliliter aliquots of the neutral $\Delta$4rbST solution are combined with an equal volume of either 2.4 mM $ZnCl_2$ or 2.4 mM $CuCl_2$. After one hour of stirring at room temperature, the suspensions are pelleted by a 30 minute, 15,000 × g centrifugation. Each pellet is frozen at -80 C and lyophilized along with a shell frozen control sample composed of 20 ml of the neutral pH $\Delta$4rbST solution diluted 50% in $dH_2O$.

The dry weight and the percentage purities of the lyophilized samples are determined to allow calculation of the efficiency of each precipitation method relative to the non-precipitated, lyophilized treatment.

To determine the effect of the metal salts on the bioactivity of $\Delta$4rbST, a growth assay is performed using hypophysectomized rats as in Example III. To conduct the test young (36 days old at the beginning of the test period), female, hypophysectomized rats are divided into groups of 10 rats. The rats are grouped by weight, the weight and distribution of the groups are balanced such that the mean starting weight of each group is nearly equal. Each group of rats is assigned a treatment.

The rats are injected subcutaneously for each of nine days with either a somatotropin or control solution. The injections are made below the skull near the subscapular region. To solubilize somatotropin the following Parlow's buffer solutions are used.

     I. $NaHCO_3$ (0.03M), NaCl (0.15M), pH raised to 10.8 with 8M NaOH.
     II. $NaHCO_3$ (0.03M), NaCl (0.15M), pH raised to 9.5 with 8M NaOH.

A known amount of the $\Delta$4rbST is dissolved in pH 10.8 buffer, adjusted to pH 9.5 with 2N HCl and made to volume with pH 9.5 buffer.

One group of 10 rats is used as a negative control and injected with the vehicle used in solubilizing the somatotropin. A second group is injected with a solution of lyophilized $\Delta$4rbST. The other groups are injected with solutions of a selected dose of transition metal-precipitation $\Delta$4rbST of this invention. The rats are weighed on days 1, 2, 9 and 10 and their body weights recorded. At the end of the test period (day 10) the rat body weight gain data is analyzed.

## EXAMPLE VI

### Precipitation of Natural Bovine Somatotropin

Purified lyophilized bovine somatotropin (bST), originally obtained from pituitaries, is reconstituted in dionized water ($dH_2O$) to a concentration of 10 mg bST per ml. The material is dialyzed against > 100 volumes of carbonate buffer (as defined in Example I) and a portion of the dialyzed material is diluted in nine parts of carbonate buffer, pH 7.4. The resultant solutions of bST then are spiked with $^{125}$I-bST to a final concentration of 0.02$\mu$ Curie per ml to allow determination of the percentage precipitation by measurement of the radioactivity in the supernatant fraction following centrifugation.

The transition metal salts used include zinc chloride, manganese chloride and cupric chloride. 24 mM, 2.4 mM and 0.24 mM solutions of each salt are made. Trichloroacetic acid (20%) is used as the positive precipitation control. The metal salts or trichloroacetic acid solutions are added to the aqueous solutions containing bST and the precipitation step is carried out with stirring for one hour at room temperature. The precipitated material is pelleted by 25 centrifugation at 15,000 × g for 10 minutes at room remperature. Single 0.5 ml aliquots of supernatant are removed from duplicate tubes and counted in polypropylene tubes. The minimum number of counts is four times over background. Pellet formation is judged visually. The 30 results of the experiments are similar to those in Example I.

## EXAMPLE VII

### Solubility and Bioactivity of Precipitated bST

In preparation for the precipitation experiment, 500 mg of bST prepared as in Example VI is reconstituted in 50 ml of de-ionized water ($dH_2O$). The material is extensively dialyzed against carbonate buffer, pH 7.4, and the solution is clarified by a 1500 × g centrifugation at room temperature.

Ten milliliter aliquots of the neutral bST solution is combined with an equal volume of either 2.4 mM $ZnCl_2$ or 2.4 mM $CuCl_2$. After one hour of stirring at room temperature, the suspensions are pelleted by a 30 minute, 15,000 × g centrifugation. Each pellet is frozen at -80 C and lyophilized along with a shell frozen control sample composed of 20 ml of the neutral pH bST solution diluted 50% in $dH_2O$.

The dry weight and the percentage purities of the lyophilized samples are determined to allow calculation of the efficiency of each precipitation method relative to the non-precipitated, lyophilized treatment.

To determine the effect of the metal salts on the bioactivity of bST, a growth assay is performed using hypophysectomized rats as in Example III.

To conduct the test, young (36 days old at the beginning of the test period), female, hypophysectomized rats are divided into groups of 10 rats. The rats are grouped by weight, the weight and distribution of the groups.are balanced such that the mean starting weight of each group is nearly equal. Each group of rats is assigned a treatment.

The rats are injected subcutaneously for each of nine days with either a somatotropin or control solution. The injections are made below the skull near the subscapular region. To solubilize somatotropin the following Parlow's buffer solutions are used.

8

I. NaHCO$_3$ (0.03M), NaCl (0.15M), pH raised to 10.8 with 8M NaOH.

II. NaHCO$_3$ (0.03M), NaCl (0.15M), pH raised to 9.5 with 8M NaOH.

A known amount of the bST is dissolved in pH 10.8 buffer, adjusted to pH 9.5 with 2N HCl and made to volume with pH 9.5 buffer.

One group of 10 rats is used as a negative control and injected with the vehicle used in solubilizing the somatotropin. A second group is injected with a solution of lyophilized bovine somatotropin. The other groups are injected with solutions of a selected dose of transition metal-precipitated bST of this invention. The rats are weighed on days 1, 2, 9 and 10 and their body weights recorded. At the end of the test period (day 10) the rat body weight gain data is analyzed.

## EXAMPLE VIII

### Precipitation of Natural Porcine Somatotropin

Purified lyophilized porcine somatotropin (pST), originally obtained from pituitaries, is reconstituted in dionized water (dH$_2$O) to a concentration of 10 mg pST per ml. The material is dialyzed against > 100 volumes of carbonate buffer (as defined in Example I) and a portion of the dialyzed material is diluted in nine parts of carbonate buffer, pH 7.4. The resultant solutions of pST then are spiked with $^{125}$I-pST to a final concentration of 0.02$\mu$ Curie per ml to allow determination of the percentage precipitation by measurement of the radioactivity in the supernatant fraction following centrifugation.

The transition metal salts used include zinc chloride, manganese chloride and cupric chloride. 24 mM, 2.4 mM and 0.24 mM solutions of each salt are made. Trichloroacetic acid (20%) is used as the positive precipitation control. The metal salts or trichloroacetic acid solutions are added to the aqueous solutions containing pST and the precipitation step is carried out with stirring for one hour at room temperature. The precipitated material is pelleted by centrifugation at 15,000 $\times$ g for 10 minutes at room remperature. Single 0.5 ml aliquots of supernatant are removed from duplicate tubes and counted in polypropylene tubes. The minimum number of counts is four times over background. Pellet formation is judged visually. The results of the experiments are similar to those in Example I.

## EXAMPLE IX

### Solubility and Bioactivity of Precipitated pST

In preparation for the precipitation experiment, 500 mg of pST prepared as in Example VIII is reconstituted in 50 ml of de-ionized water (dH$_2$0). The material is extensively dialyzed against carbonate buffer, pH 7.4, and the solution is clarified by a 1500 $\times$ g centrifugation at room temperature.

Ten milliliter aliquots of the neutral pST solution. is combined with an equal volume of either 2.4 mM ZnCl$_2$ or 2.4 mM CuCl$_2$. After one hour of stirring at room temperature, the suspensions are pelleted by a 30 minute, 15,000 $\times$ g centrifugation. Each pellet is frozen at -80 C and lyophilized along with a shell frozen control sample composed of 20 ml of the neutral pH pST solution diluted 50% in dH$_2$O.

The dry weight and the percentage purities of the lyophilized samples are determined to allow calculation of the efficiency of each precipitation method relative to the non-precipitated, lyophilized treatment.

To determine the effect of the metal salts on the bioactivity of pST, a growth assay is performed using hypophysectomized rats as in Example III.

To conduct the test, young (36 days old at the beginning of the test period), female, hypophysectomized rats are divided into groups of 10 rats. The rats are grouped by weight, the weight and distribution of the groups are balanced such that the mean starting weight of each group is nearly equal. Each group of rats is assigned a treatment.

The rats are injected subcutaneously for each of nine days with either a somatotropin or control solution. The injections are made below the skull near the subscapular region. To solubilize somatotropin the following Parlow's buffer solutions are used.

I. NaHCO$_3$ (0.03M), NaCl (0.15M), pH raised to 10.8 with 8M NaOH.

II. NaHCO$_3$ (0.03M), NaCl (0.15M), pH raised to 9.5 with 8M NaOH.

A known amount of the pST is dissolved in pH 10.8 buffer, adjusted to pH 9.5 with 2N HCl and made to volume with pH 9.5 buffer.

One group of 10 rats is used as a negative control and injected with the vehicle used in solubilizing the somatotropin. A second group is injected with a solution of lyophilized porcine somatotropin. The other groups are injected with solutions of a selected dose of transition metal-precipitated pST of this invention. The rats are weighed on days 1, 2, 9 and 10 and their body weights recorded. At the end of the test period (day 10) the rat body weight gain data is analyzed.

## EXAMPLE X

Forty-three liters of purified Δ7rpST at a concentration of 590 mg/1 in 60 mM ethanolamine buffer, pH 9.0 was obtained from E. coli HB101 which had been grown in a fermentation medium. The solution was concentrated to about 810 mg/1 in a cross-flow ultrafiltration unit, Romicon HF, equipped with one 26-ft$^3$ membrane cartridge that had a nominal molecular weight cut-off of approximately 5,000 daltons. The resulting concentrated product solution in the ethanolamine buffer at pH 9.0 was diafiltered in the same UF unit against 6 × volumes of 50% carbonate buffer (0.21 mM $Na_2CO_3$; 0.25 mM $NaHCO_3$) at pH 8.0 to 10.0. The resulting product retentate, now in 50% carbonate buffer with pH above 8.0, turned slightly cloudy ($OD_{595}$ = 0.01) and was clarified in a cross-flow microporous filtration unit, Amicon DC-10, equipped with one 5-$ft^2$ membrane cartridge having 0.1 μm membrane.

The above clarified solution, 26 liter at 800 mg/1 ST concentration and pH 9.0, was gently mixed and added with 3.7 liter of 0.2 μ-filtered 2.4 mM $ZnCl_2$ solution at a rate that the addition would be completed in a period of 5 to 30 minutes. The amount of Zn++ addition in this experiment resulted in Zn/ST molar ratio of approximately 10. Before further recovery of the Zn-Δ7rpST precipitate, the solution was mixed for another 20 minutes.

The resulting ZnΔ7rpST suspension, now at 0.08% concentration with particle sizes 2-5 μm, was concentrated to approximately 2-5% in a cross-flow microporous filtration unit with 0.1 μ membrane cut-off.

The resulting suspension was further concentrated to about 250,000 mg/1 by continuous bowl-type centrifugation. Alternatively, the resulting suspension can be directly spray-dried or can be further concentrated to about 100,000 mg/1 by either cross-flow microporous filtration or disc-stack centrifugation. The resulting paste was then spread to a thickness of about 2 cm and freeze-dried for two days with the maximum product temperature limited to 25°C. The freeze-dried material that generally had a particle size of 10-200 μm and a bulk density of approximately 0.5 is suitable for direct formulation into a delivery system.

EXAMPLE XI

Example X was repeated except that the 50% carbonate buffer was substituted by 2 mM Tris buffer at pH 7.4. The final product characteristics and precipitation efficiency were similar between Tris and 50% carbonate buffer.

**Claims**

1. A method of recovering somatotropin from dilute aqueous solution comprising adding to the aqueous solution a salt of a transition metal in a sufficient amount to form an insoluble complex with the somatotropin and separating the complex from the solution.

2. The method of claim 1 wherein the somatotropin is a biologically active fragment of somatotropin.

3. The method of claim 2 wherein the somatotropin is a recombinant biologically active fragment of somatotropin.

4. The method of claim 3 wherein the somatotropin is porcine somatotropin.

5. The method of claim 4, wherein the somatotropin is a biologically active fragment of porcine somatotropin in which the first seven amino-terminal amino acids of the mature protein have been deleted.

6. The method of claim 3 wherein the somatotropin is a recombinant biologically active fragment of bovine somatotropin.

7. The method of claim 6, wherein the somatotropin is a biologically active fragment of bovine somatotropin in which the first four amino-terminal acids of the mature protein have been deleted

8. The method of any of claims 1 to 7 wherein the metal comprises zinc, copper, manganese, iron or cobalt.

9. The method of claim 8 wherein the metal is zinc.

10. The method of any of claims 1 to 9 wherein the transition metal salt comprises a chloride, sulphate, acetate, or tartrate of a transition metal.

11. The method of claim 10 wherein the salt is zinc chloride.

12. The method of any of claims 1 to 11 wherein the insoluble complex is separated from the aqueous solution by filtration or centrifugation and dried.

13. The method of any of claims 1 to 12 wherein the dilute aqueous solution is at a pH between about 6.8 and about 9.8 and there is a molar excess of the salt of the transition metal over the amount of somatotropin.

14. The method of any of claims 1 to 13 wherein the concentration of transition metal salt is at least about 0.12 mmoles per 0.025 mmoles of somatotropin in 1.0 litre solution.

15. The method of any of claims 1 to 14 wherein the resultant insoluble complex contains at least about 1 to about 8 moles metal per mole somatotropin.

16. The method of any of claims 1 to 14 wherein the resultant insoluble complex contains from about 0.3 to about 8 percent by weight transition metal.

17. The method of any of claims 1 to 16 wherein the dilute aqueous solution contains a buffer comprising carbonate buffer, 50 mM Tris HCl, or 60 mM ethanolamine.

18. Biologically active somatotropin in the form of a complex with a transition metal.

19. A biologically active fragment of somatotropin in the form of a complex with a transition metal.

20. The use of a complex as claimed in claim 18 or claim 19 for the preparation of a growth enhancing pharmaceutical or veterinary composition.